# EUROPEAN PATENT APPLICATION

(11) **EP 3 354 266 A1**
(43) Date of publication of application: **01.08.2018**
(21) Application number: 16848188.5
(22) Date of filing: 19.09.2016
(51) Int. Cl.: A61K 31/5415, A61P 25/18

(54) **AMINOPHENOTHIAZINES FOR MODULATING THE NUMBER OF SYNAPSES**

(30) Priority: 23.09.2015 ES 201531358
(71) Applicant: Consejo Superior De Investigaciones Científicas (CSIC), 28006 Madrid (ES)
(72) Inventor: MARTÍNEZ GIL, Ana, 28040 Madrid (ES); GIL AYUSO-GONTAN, Carmen, 28040 Madrid (ES); CAMPILLO MARTÍN, Nuria, Madrid 28040 (ES); SANCHEZ BARRENA, Mª José, 28006 Madrid (ES); MANSILLA APARICIO, Alicia, 28002 Madrid (ES); FERRÚS GAMERO, Alberto, 28002 Madrid (ES)
(74) Representative: Pons
(86) International application number: PCT/ES2016/070649
(87) International publication number: WO 2017/051046

(57) **Abstract**

The invention relates to the use of a group of compounds with a phenothiazine nucleus of general formula (I) for the treatment of diseases of the central nervous system which present synapse abnormalities, such as autism or fragile X syndrome. The compounds according to the invention intervene in the interaction of the NCS1 and Ric8 proteins involved in the process of synaptogenesis.

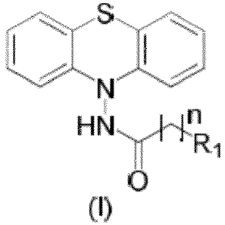

## Description

The present invention is encompassed within the sector of pharmaceutical chemistry and relates to the use of a group of compounds with a phenothiazine core for treating diseases of the central nervous system which present abnormalities in the synapse number. The compounds of the invention are involved in the interaction of the NCS1 and Ric8 proteins involved in the process of regulating the synapse number and the probability of releasing neurotransmitter.

### STATE OF THE ART

Synaptogenesis is an active maturation process which comprises the formation of a locus that releases neurotransmitters in the presynaptic neuron and an associated postsynaptic receiver field as well as the precise alignment of the presynaptic and postsynaptic specializations. The suitable balance between excitatory and inhibitory synapse is basic during the development of the neuronal circuits. The mutations in the genes, which regulate this balance, cause various neurodevelopmental disorders such as autism, epilepsy, Angelman syndrome, Fragile X syndrome and Rett syndrome. These mutations may cause deviations in the normal synapse number and in the functional properties thereof.

In the document WO2009/156535, a group of hydrazides is described which include the compound FD44 and the therapeutic use thereof for treating SNC diseases where a deficit or dysfunction of the cholinergic neurotransmission is produced such as for example Alzheimer's, Parkinson or Huntington.

In the documents WO0192240 and European Journal of Medicinal Chemistry 45 (2010) 6152-6158, a series of N-substituted phenothiazines and the use thereof for treating Alzheimer's is described.

### DESCRIPTION OF THE INVENTION

In a first aspect, the present invention relates to the use of a compound of formula (I): 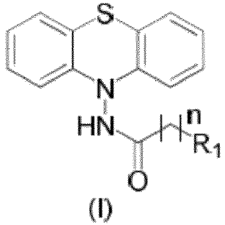 wherein R₁ is selected from C₁-C₄ alkyl, C₃-C₆ heterocycle, aryl, heteroaryl, - COOH, -NR₂R₃, R₂ and R₃ being independently selected from H, alkyl or aryl and n is a value between 1 and 4 or any of the pharmaceutically acceptable salts, isomers or solvates thereof for manufacturing a medicament for treating diseases of the central nervous system which present abnormalities in the synapse.

In a preferred embodiment, n is 1.

In another preferred embodiment, R₁ is heterocycle. In a more preferred embodiment, R₁ is selected from piperidine or pyrrolidine.

In another preferred embodiment, the compound of formula (I) is selected from the following compounds:

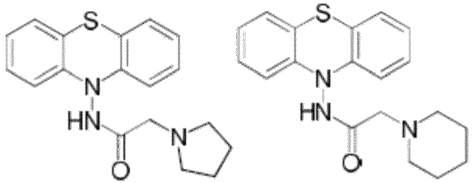

In another preferred embodiment, the diseases of the central nervous system which present abnormalities in the synapse are selected from autism, Fragile X syndrome, attention deficit disorder, epilepsy, Angelman syndrome, Rett syndrome or schizophrenia.

In a more preferred embodiment, the disease of the central nervous system which presents abnormalities in the synapse is autism.

In another more preferred embodiment, the disease of the central nervous system which presents abnormalities in the synapse is Fragile X syndrome.

The protein complex formed by the neuronal calcium sensor 1 (NCS1) and the guanine exchange factor Ric8 regulates the synapse number and the probability of neurotransmitter release, consequently it can be considered a therapeutic target for treating diseases in which the synapse is affected. The structural identity between NCS-1 and Frq2 (its homologous protein in Drosophila) is very high, a specific interaction between Frq2 and Ric8 having been reported. From the structural data of the interaction surface between Frq2 and Ric8 obtained from their crystalline complex, a set of phenothiazines with formula (I) capable of modulating this interaction have been identified by virtual screening. Therefore, these phenothiazines may be considered as candidates for drugs for treating various synaptopathies.

The term "alkyl" in the present invention relates to linear or branched hydrocarbon chain radicals which have 1 to 10 carbon atoms, preferably 1 to 6 and more preferably 1 to 4 and which are bonded to the rest of the molecule by means of a single bond, for example, propyl, ethyl, methyl, isopropyl, undecanoyl, heptadecanoyl, octadecanoyl, etc. These alkyl radicals may be optionally substituted in one or more positions by one or more groups such as hydroxyl, amines, amides, oxo, cyano, halogens, aryl, etc.

The term, "aryl" in the present invention relates to single or multiple aromatic rings which have between 5 and 18 bonds in which one proton has been eliminated from the ring. The aryl group preferably has 5 to 7 carbon atoms. The aryl groups are for example, but not limited to, phenyl, naphthyl, diphenyl, indenyl, phenanthryl or antracyl. The aryl radicals may be optionally substituted by one or more substituents such as alkyl, hydroxyl, amines, amides, cyano, halogens, etc.

The term, "heteroaryl" in the present invention relates to an aryl group which contains at least one heteroatom.

The term, "heterocycle" in the present invention relates to cyclic hydrocarbon chains which have 3 to 6 carbon atoms and which are bonded to the rest of the molecule by means of a single bond in which at least one carbon atom of the main chain has been substituted by O, N or S. They can be saturated or unsaturated although they are preferably saturated such as for example piperidine, pyrrolidine, tetrahydrofuran, tetrahydrothiophene, etc. These heterocycle radicals may be optionally substituted in one or more positions by one or more groups such as hydroxyl, amines, amides, oxo, cyano, halogens, aryl, etc.

The compounds of the present invention represented by the formula (I) and more specifically the specific compounds belonging to this previously described general formula may include isomers, depending on the presence of multiple bonds (for example Z, E), including optic isomers or enantiomers, depending on the presence of chiral centers. The individual isomers, enantiomers or diastereoisomers and the mixtures of the same fall within the scope of the present invention. The individual enantiomers or diastereoisomers as well as their mixtures may be separated by means of conventional techniques.

The compounds of the invention may be in crystalline form like free compounds or like solvates and it is intended for both forms to be within the scope of the present invention. In this sense, the term, "solvate" as it is used here, includes both pharmaceutically acceptable solvates, that is to say, solvates of the compound of formula (I) which may be used in the preparation of a medication, and pharmaceutically unacceptable solvates which may be used in the preparation of pharmaceutically acceptable solvates or salts. The nature of the pharmaceutically acceptable solvate is not critical provided it is pharmaceutically acceptable. In a particular embodiment, the solvate is a hydrate. The solvates may be obtained by means of conventional methods of solvation, well known by persons skilled in the art.

For its therapeutic application, the compounds of formula (I), their isomers, salts or solvates are preferably in a pharmaceutically acceptable or substantially pure form, that is to say, it has a pharmaceutically acceptable level of purity excluding the normal pharmaceutical additives such as diluents and carriers and not including material considered toxic at normal dosing levels. The purity levels for the active ingredient are preferably greater than 50%, more preferably greater than 70%, more preferably greater than 90%. In a preferred embodiment, they are greater than 95% of the compound of formula (I) or isomers, salts or solvates thereof.

Unless otherwise indicated, the compounds of the invention also include compounds which differ only in the presence of one or more isotopically enriched atoms. For example, compounds which have said structure, with the exception of the substitution of a hydrogen for a deuterium or for tritium, or the substitution of a carbon for a ¹³C or ¹⁴C enriched carbon or a ¹⁵N enriched nitrogen, they are within the scope of this invention.

The compounds of formula (I) for the therapeutic use thereof are prepared in solid form or aqueous suspension in a pharmaceutically acceptable diluent. These preparations may be administered by any suitable route of administration for which said preparation is formulated in the pharmaceutical form suitable for the selected route of administration. For example, the compounds of formula (I) are combined with excipients such as starch or lactose or coadjuvants such as cyclodextrins as well as any type of pharmaceutical carriers known by the person skilled in the art for preparing solid or liquid formulations. In a particular embodiment, the administration of the compound of formula (I) provided by this invention is effected by oral, topical, rectal or parenteral route (including subcutaneous, intraperitoneal, intradermic, intramuscular, intravenous, etc.). A review of the different pharmaceutical forms of administration of medications and of the excipients required for obtaining the same may be found, for example in "Tratado de Farmacia Galénica", C. Faulí i Trillo, 1993, Luzán 5, S.A. Ediciones, Madrid, or in other conventional or similar publications of Spanish, European or US pharmacopeias.

The compounds described in the present invention, the pharmaceutically acceptable salts and solvates thereof and the pharmaceutical compositions which they contain may be used together with other additional drugs to provide a combination therapy. Said additional drugs may form part of the same pharmaceutical composition or alternatively, may be provided in the form of a separate composition for administering it simultaneously or non-simultaneously to the administration of the pharmaceutical composition which comprises a compound of formula (I) or an isomer, solvate or a pharmaceutically acceptable salt of the same.

Another aspect of the invention relates to a method for modulating the synapse number which comprises the administration of a therapeutically effective amount of a compound of formula (I) as previously described.

Within the context used in this description, the expression "therapeutically effective amount" relates to the quantity of active compound sufficient for producing the desired effect in which the symptoms of the disease are attenuated. The dose should not be used in proportions which cause undesired collateral effects, the clinical valuation of which makes them adverse and not therapeutically treatable. The dose generally varies with age, status, sex and extent of the disease in the patient as well as the route and frequency of administration and can be determined in each case.

The use of the compounds of the invention is compatible with their use in protocols in which the compounds of formula (I) or the mixtures thereof are used by themselves or in combinations with other treatments or any medical procedure.

Throughout the description and the claims, the word "comprises" and the variants thereof do not intend to exclude other technical characteristics, additives, components or steps. Other objects, advantages and characteristics of the invention will emerge for the person skilled in the art in part from the description and in part from the practice of the invention. The following examples and figures are provided for illustration and are not intended to limit the present invention.

### BRIEF DESCRIPTION OF THE FIGURES

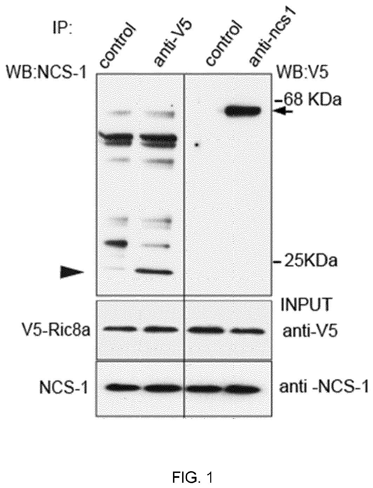

**Fig. 1** shows the results of the coimmunoprecipitation (coIP) assay between human NCS1 and Ric8a. Left panel = precipitation with anti-V5 which is bonded to Ric8a, following by immunodetection in Western blot of NCS1 with a specific antibody. Right panel = precipitation with anti-NCS1, followed by immunodetection of Ric8a in Western blot with anti-V5 antibody. Lower panels = load input in the corresponding gels (1/10 of the protein lysate prior to immunoprecipitation).

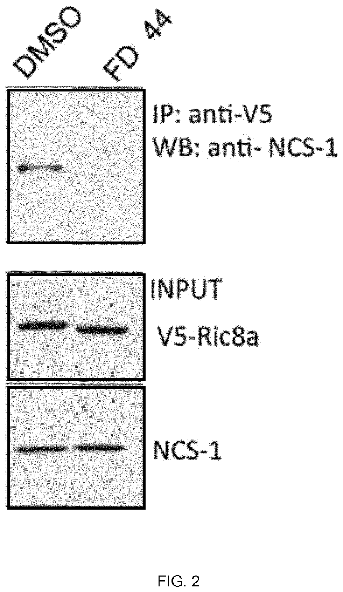

**Fig. 2** shows the results of the immunoprecipitation assays in HEK293 cells transfected with human V5-Ric8a and NCS1 and in the presence or otherwise of the compounds. The DMSO lane represents a positive control of the interaction.

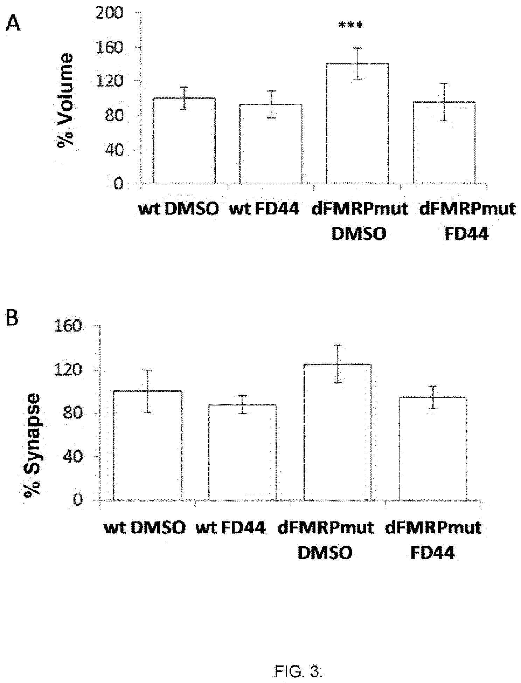

**Fig. 3 A)** volume of the neuromuscular plate normalized with respect to the wild type. Wt DMSO = wild type larvae grown in medium with DMSO carrier (200 micromolar), wt FD44 = larvae grown in medium with FD44 dissolved in DMSO (200 micromolar), dFMRPmutDMSO = mutant larvae for the *fmr1* gene grown in medium with DMSO, dFMRPmut FD44 = mutant larvae grown in medium with FD44 (200 micromolar). B) Same genotypes computing the synapse number (immunopositive points for the nc82 monoclonal antibody). **C)** Same genotypes computing the number of collateral branches. **D)** Representative images of each genotype. Each image is an accumulation of all the confocal planes.

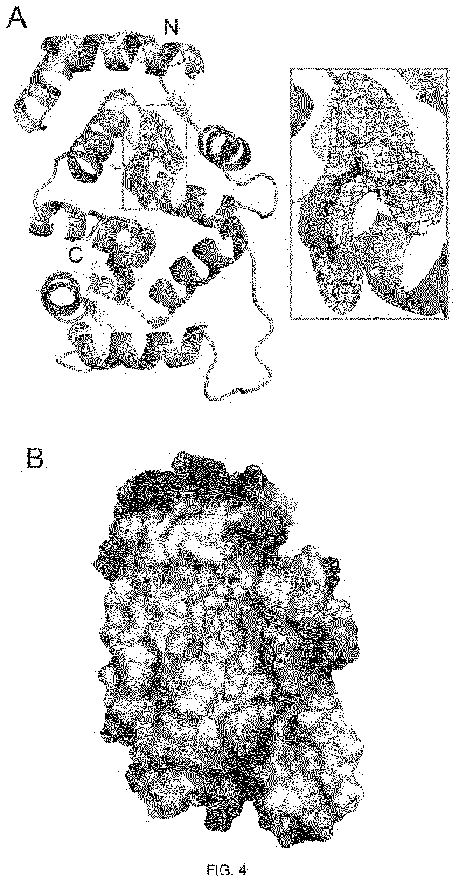

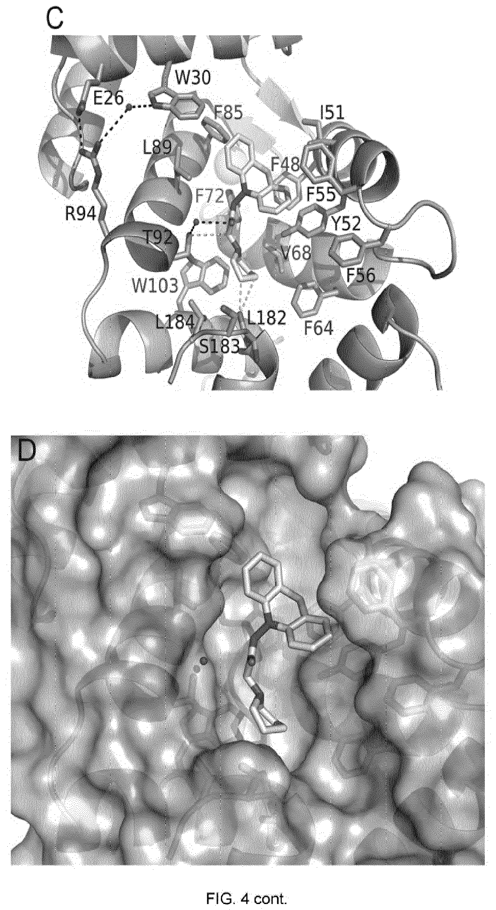

**Fig. 4** (A) Representation of the crystallographic structure of the NCS1/FD44 complex. The FD44 molecule is shown in sticks. The 2Fo-Fc map of the small molecule is shown at 1σ. (B) Representation of the electrostatic molecular surface of NCS1 bound to FD44 where the hydrophobic environment of FD44 is observed. (C) Detailed view of the amino acids in contact with FD44. The majority of the interactions are Van der Waals, except a strong hydrogen bond with T92, that is mediated by water molecule (with discontinuous black lines), and three weak hydrogen bonds where a C atom is the hydrogen donor and an O atom is the acceptor. (D) Close-up view of the cavity where FD44 is bound.

### EXAMPLES

The invention is illustrated below by means of tests carried out by the inventors which reveal the efficacy of the product of the invention.

### Example 1. Virtual screening in Frq2

The FD44 compound was identified by means of a virtual test ("virtual screening") using the own library of the group and the human NCS-1 structure (pdb 1g8i code, Bourne et al., 2001 J Biol Chem. 13;276(15):11949-55.) and of Drosophila (pdb 4by4 code, Romero-Pozuelo et al., 2014. J Cell Sci. 127, 4246-4259). The virtual screening was carried out using the Glide program (Maestro Version 9.4.047, Schrodinger® Software).

The molecules, 32 phenothiazines, were extracted from the library of the group. These molecules were prepared using the Maestro Ligprep wizard module (Schrödinger® Software Modules). The hydrogens were added and were minimized using the OPLS_2005 force field. Lastly, 10 formations were generated for each ligand using Glide. After preparing the library and the protein, a grid was defined centered on the Arg94 amino acid. The docking studies were carried out with the Glide standard precision module. The possible ways of bonding that were generated were classified both using the Gscore scoring function generated by Glide and as a function of the interactions which were established with the Arg94 key residue. For the analysis of the interactions of the protein-ligand complexes, the Liginteractions module was used, implemented in Maestro (Maestro Version 9.4.047, Schrödinger® Software).

### Example 2: Maintaining the interaction between NCS1 and Ric8a in humans.

In order to check whether the interaction which has been previously detected (Romero- Pozuelo J et al. The guanine-exchange factor Ric8a binds to the Ca2+ sensor NCS-1 to regulate synapse number and neurotransmitter release. J Cell Sci. 2014 Oct 1;127(Pt 19):4246-59) between the Drosophila Frq2 and Ric8a proteins, the coimmunoprecipitation of their human equivalents, NCS1 and Ric8a was tested. Human Ric8a was subcloned into nV5pCDNA3.1. The NCS1 construct was subcloned into pCDNA3.1. The cotransfections were carried out in HEK293 cells using the Superfect® (Qiagen®) reagent and lysed 48 hours later. Before its clarification, the lysates were incubated with the respective compound at 4° C for the night (12 hours) with and without anti-V5 (Invitrogen®) or anti-NCS1 (Cell Signaling Technology®) antibodies as is indicated in each experiment. As a negative control, immunoprecipitations were carried out with an unrelated antibody. The following antibodies and dilutions were used with the Western blots: rabbit anti-NCS1 (1:1000) and mouse anti-V5 (1:2000). Following cotransfection of HEK293 cells of both constructs, the data show that the human homologues maintain the interaction (Fig. 1). The Ric8a immunoprecipitation pulls NCS1 and vice versa. These data show that the Drosophila model may be used as an *in vivo* model of the interaction of NCS-1 and Ric8.

### Example 3: Interference tests regarding the interaction of NCS1/Ric8a in vitro.

With the aim of evaluating the capacity of the derivatives FD44 to inhibit the interaction between human NCS1 and Ric8a, HEK293 cells were transfected with a version of Ric8a marked with the V5 epitope and with NCS1, the cellular lysate being incubated with the product to be tested. The data (Fig. 2) indicate that FD44 effectively inhibits this interaction.

### Example 4: Synaptic effects of FD44

The effect of FD44 on the synapse number *in vivo* was determined using transgenic Drosophila for the fmr1 gene, administering FD44 in the intake.

The mutating in the *fmr1* gene corresponds to Fmr1^{Δ50M} # BL-6930 and the wild type corresponds to the Canton-S strain, all originating from the repository of the Bloomington Drosophila Stock Center. The FD44 compound was dissolved in DMSO and added to the Drosophila culture medium in fluid phase to ensure the homogenous distribution thereof resulting in a final concentration of 200 micromolar. Once the medium had cooled, the vials were used normally for placing eggs and growing larvae at 25°C until reaching the advanced LIII stage.

Reaching the advanced larva III stage, the animals were surgically processed to expose the neuromuscular junctures (NMJ) following the described method (Martin-Peña et al., Age-independent synaptogenesis by phosphoinositide 3 kinase. J Neurosci. 2006 Oct 4;26(40):10199-208). The nc82 antibody is a specific monoclonal for the Bruchpilot protein of Drosophila belonging to the CAST family of vertebrae and forms the presynaptic specialization. It was obtained from the Drosophila Hybridoma Bank (DSHB). The anti-HRP antibody produced in rabbits marks the neuronal membrane and was obtained from Jackson ImmunoResearch. A larval neuromuscular juncture was analyzed up to a total of 8-12 larvae per genotype to avoid growth artefacts. The serialized confocal sections of 1 micrometer thickness were analyzed and quantified using the Imaris® program.

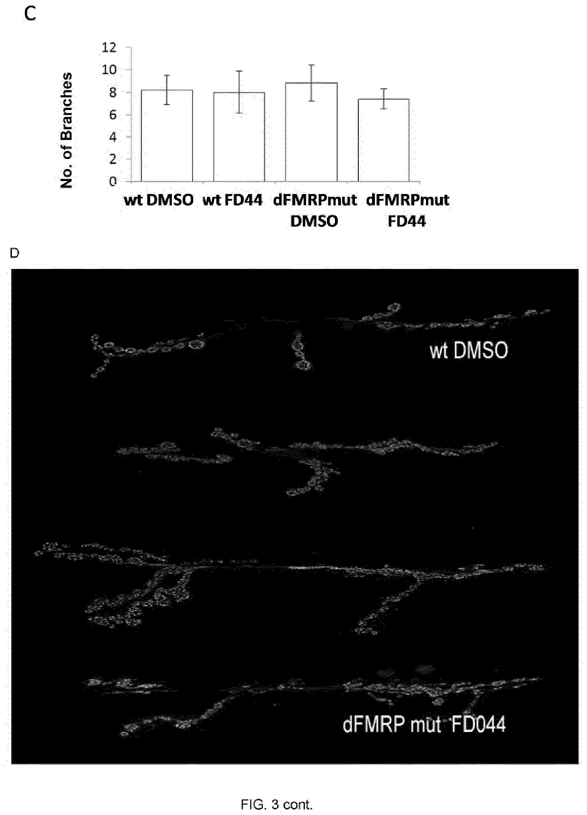

In order to measure the FD44 effects administrated via intake, the neuromuscular juncture corresponding to the motor neuron 6/7 of the A3 larval abdominal segment was used. The larval preparations were stained with the nc82 antibody to visualize the synapse and with the anti-HRP to visualize the membrane of the axon. Lastly, the synapse number was measured in images obtained in confocal microscopy and quantified by means of Imaris® software. The data show (Fig. 1) that the total volume of the motor neutron is significantly increased in the case of the mutant *frm1* as has been previously described (Morales et al., Drosophila fragile X protein, DFXR, regulates neuronal morphology and function in the brain. Neuron. 2002 34(6):961-72). Moreover, the administration of FD44 or the diluent, DMSO, are innocuous for the wild type control (Fig. 3A). However, the administration of FD44 to the mutant, reverts the neuronal projection volume to the normal levels of the controls (Fig. 3A). The synapse number experiences the same excess effect in the mutant and reversion in the mutant which has ingested FD44 (Fig. 3B). Lastly, the collateral branch number does not show any change in the different genotypes and intake types, indicating that the neuritogenesis is not affected by the mutant or FD44 (Fig. 3C). In other words, the mutant and the treatment affect the axonal growth and the synaptogenesis, but not the neuritogenesis. This presents an advantage for FD44 in terms of a potential treatment since it should not cause collateral effects, possibly harmful, when the branches (neurites), which could have established abnormal connections, are modified. Representative images of the main genotypes are shown in Fig. 3D.

### Example 5: Interaction of the FD44 compound with the NCS1 protein

The purified Drosophila NCS-1 (Frq2) protein was dialyzed with water and concentrated to 10 mg ml-1 using a concentrator with a cut membrane of 10 kDa (Vivaspin®). 0.3 mg of FD44 were added to 250 µl of protein sample and incubated for 16 hours at 4°C. The insoluble fraction of the compound was separated by centrifugation. The preliminary crystallization experiments were carried out using the Innovadine® crystallization robot and commercial crystallization kits (Molecular Dimensions®, Qiagen®, Hampton Research® and Jena Bioscience®). The mixture of 1 µl of protein previously incubated with FD44, with 1 µl of the A11 solution of Proplex (0.1 M Hepes pH 7.5 and 25% w/v/ of PEG 2,000 MME) to which 1.7 mM of FD44 had been previously added produced good quality monocrystals. These crystals were cryoprotected with a solution which contained 15% of glycerol, 0.1 M Hepes pH 7.5, 25% w/v/ of PEG 2,000 MME and 0.5 mM FD44 and were subsequently cooled down in liquid nitrogen. A 1.6 Å diffraction dataset was collected at ALBA synchrotron (BL13 beamline) and belonged to the space group P2₁2₁2₁. The data were processed with XDS and the structure was solved using the molecular replacement method with Phaser and using the structure of the apoprotein as a starting model. A molecule was found in the asymmetric unit. The structure was refined with Phenix.

In Figure 4, the crystallographic structure of the NCS-1 complex with FD44 is shown. This complex explains the biological activity of FD44: the compound is inserted in the N-terminal part of the elongated hydrophobic groove that the protein presents and stabilizes the C-terminal helix H10 within the same. All of this means that the hydrophobic groove, interaction surface for Ric8, is completely blocked and inaccessible.

## Claims

1. A use of a compound of formula (I): 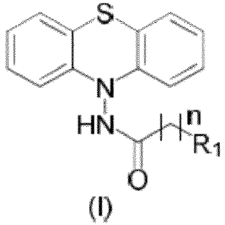 wherein R₁ is selected from C₁-C₄ alkyl, C₃-C₆ heterocycle, aryl, heteroaryl, -COOH, - NR₂R₃, R₂ and R₃ being independently selected from H, alkyl or aryl and n is a value between 1 and 4 or any of the pharmaceutically acceptable salts, isomers or solvates thereof for manufacturing a medication for treating diseases of the central nervous system which present abnormalities in the synapse.

2. The use of the compound according to the preceding claim wherein n is 1.

3. The use of a compound according to any of the preceding claims wherein R₁ is heterocycle.

4. The use according to the preceding claim wherein R₁ is selected from piperidine or pyrrolidine.

5. The use according to any of the preceding claims wherein the compound of formula (I) is selected from the following components:

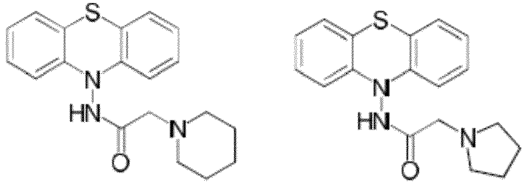

6. The use according to any of the preceding claims wherein the diseases of the central nervous system which present abnormalities in the synapse are selected from autism, Fragile X syndrome, attention deficit disorder, epilepsy, Angelman syndrome, Rett syndrome or schizophrenia.

7. The use according to the preceding claim wherein the disease of the central nervous system which presents abnormalities in the synapse is autism.

8. The use according to claim 6 wherein the disease of the central nervous system which presents abnormalities in the synapse is Fragile X syndrome.
